# EUROPEAN PATENT SPECIFICATION

(11) **EP 2 289 414 B1**
(45) Date of publication and mention of the grant of the patent: **02.10.2019**
(21) Application number: 10174673.3
(22) Date of filing: 31.08.2010
(51) Int. Cl.: A61B 5/113

(54) **Method and system for limiting interference in magnetometer fields**
Verfahren und System zur Eingrenzung der Interferenz in Magnetometerfeldern
Procédé et système pour limiter l'interférence dans des champs de magnétomètre

(30) Priority: 01.09.2009 US 275576 P; 26.08.2010 US 869576
(43) Date of publication of application: 02.03.2011
(73) Proprietor: Adidas AG, 91072 Herzogenaurach (DE)
(72) Inventor: Stone, Robert, Sunnyvale, CA 94087 (US)
(74) Representative: Wegner, Hans

(56) References cited:
- WO-A1-01/28420
- US-A- 4 258 718
- US-A- 4 267 845
- US-A1- 2002 123 701
- US-B1- 6 268 725
- US-B1- 6 373 240

## Description

### Cross-Reference to Related Applications

This non-provisional application claims priority to U.S. Provisional Application No. 61/275,576, filed on September 1, 2009.

### Field of the Invention

The present invention relates generally to magnetometer-based systems for monitoring anatomical and physiological parameters of a subject. More particularly, the invention relates to methods and systems for limiting electromagnetic interference in magnetometer fields and the resultant degradation in quality of signals reflecting changes in the magnetometer fields.

### Background of the Invention

In medical diagnosis and treatment of a subject, it is often desirable to assess one or more physiological or performance characteristics or symptoms associated with the subject. Athletic performance and progress are often evaluated by examining changes in physiological and/or performance characteristics. Respiratory air volume and other respiratory characteristics can be useful to assess athletic performance, for example, by aiding in detection of changes in physiological state and/or performance characteristics. A key respiratory characteristic is respiratory air volume (or tidal volume). Various conventional methods and systems have been employed to measure (or determine) tidal volume.

Conventional systems (and associated methods) for determining tidal volume include having the patient or subject breathe into a mouthpiece connected to a flow rate measuring device. Another system comprises a conventional respiration monitor, such as those disclosed in U.S. Patent No. 3,831,586, issued August 27, 1974, and U.S. Patent No. 4,033,332, issued July 5, 1977.

A further means for determining tidal volume is to measure the change in size (or displacement) of the rib cage and abdomen, as it is well known that lung volume is a function of these two parameters. A number of systems and devices have been employed to measure the change in size (i.e., Δ circumference) of the rib cage and abdomen, including mercury in rubber strain gauges, pneu-mobelts, respiratory inductive plethysmograph (RIP) belts, and magnetometers. See, D.L. Wade, "Movements of the Thoracic Cage and Diaphragm in Respiration", J. Physiol., pp. 124-193 (1954), Mead, et al., "Pulmonary Ventilation Measured from Body Surface Movements", Science, pp. 196, 1383-1384 (1967).

As is well known in the art, respiratory magnetometer systems typically comprise one or more tuned pairs of air-core magnetometers or electromagnetic coils. Other types of magnetometers sensitive to changes in distance therebetween can also be used. One magnetometer is adapted to transmit a specific high frequency AC magnetic field and the other magnetometer is adapted to receive the field. The paired magnetometers are responsive to changes in a spaced distance therebetween; the changes being reflected in changes in the strength of the magnetic field.

A typical respiratory magnetometer system includes a pair of magnetometers. Illustrative are the magnetometer systems disclosed in co-pending U.S. Application No. 12/231,692, filed September 5, 2008, U.S. Provisional Application No. 61/275,574, filed on September 1, 2009, and U.S. Provisional Application No. 61/275,575, filed on September 1, 2009.

To measure changes in (or displacement of) the anteroposterior diameter of the rib cage, a first magnetometer is typically placed over the sternum at the level of the 4th intercostal space and the second magnetometer is placed over the spine at the same level. Using additional magnetometers can increase the accuracy of the magnetometer system. For example, to measure changes in the anteroposterior diameter of the abdomen, a third magnetometer can be placed on the abdomen at the level of the umbilicus and a fourth magnetometer can be placed over the spine at the same level.

The signal processing techniques employed with magnetometers typically utilize fixed-frequency electromagnetic sources and conventional signal detection apparatuses (and associated methods), such as product detectors, amplitude detectors, and narrow band filters. Such sources and apparatuses are, however, susceptible to electromagnetic interference from periodic environmental sources (e.g., electrical power lines) that have a frequency synchronous with or sub-harmonic of the magnetic field frequency.

The spurious interference can, and in many instances will, result in inaccurate measurements of the magnetometer field. Anatomical displacements determined from inaccurate magnetic field measurements (e.g., signals reflecting anatomical displacements) and, hence, respiratory characteristics derived from the determined anatomical displacements can, thus, be in error.

Various conventional systems and associated techniques have thus been employed to reduce the effect of electromagnetic interference on magnetic field measurement. Such systems and techniques include conventional filtering systems having multiple filters, (e.g., a low pass statistical filter and a filter based on frequency characteristics), a signal convolved with a data dependent frequency function, such as that disclosed in U.S. Patent No. 7,295,928, issued November 13, 2007, and various time/frequency domain and Fourier techniques, such as those disclosed in co-pending U.S. Patent Application No. 10/991,877, filed November 18, 2004.

Although the noted conventional systems and techniques successfully limit interference in low to mid-noise environments, magnetometers employing the noted systems and techniques are still susceptible to interference from signal frequencies and harmonics that are close to the magnetic field frequencies.

Several additional systems and associated techniques to mitigate electromagnetic interference have been employed with flux-gate magnetometers. Such systems and techniques include differential circuitry and modifying the drive signal to minimize the probability of electromagnetic interference tracking the drive signal.

Differential circuitry techniques include the use of two magnetometers or sensors oriented opposite to one another in a magnetic field such that one sensor provides a second harmonic signal, which is inverted with respect to the other sensor. By subtracting the two signals, interference or noise is mitigated or canceled.

U.S. Patent No. 6,268,725, issued July 31, 2001, discloses systems and associated methods for reducing the effect of electromagnetic interference by modifying the flux-gate magnetometer drive signal. According to the invention, the drive signal has a time varying characteristic, which makes it unlikely that electromagnetic interference could track or mimic the drive signal, i.e., coincide with the second harmonic signal of the drive signal.

WO 01/28420 discloses an apparatus for assessing pulmonary ventilation that includes data acquisition circuitry with a first transmission coil, a second transmission coil, a first receive coil, and a second receive coil to generate pulmonary ventilation data defining three degrees of motion of an ambulatory patient. A portable data processing unit worn by the patient is connected to the data acquisition circuitry and processes the pulmonary ventilation data to produce calculated parameters.

Although the noted systems and techniques successfully limit interference in a drive signal, the systems and techniques are limited to flux-gate magnetometers. As is well known in the art, flux-gate magnetometers employ only a driving receiver. Further, only a residual magnetic field is measured.

The noted systems and techniques would thus be ineffective in reducing electromagnetic interference in received magnetic fields and, hence, the effects of such interference on magnetic field transmission measurements.

It would thus be desirable to provide an improved method and associated systems for limiting interference in magnetometer fields and/or the effects of interference on magnetic field measurements, which substantially reduce or eliminate the drawbacks and disadvantages associated with conventional methods and systems for limiting magnetometer interference.

### BRIEF SUMMARY OF THE INVENTION

The present invention provides apparatuses and methods for improved monitoring of a subject's respiratory characteristics, which is of particular use in the fields of athletic performance monitoring and medical evaluation.

The invention is a device as defined by claim 1 and a method of operating said device as defined by claim 5.

The magnetic field characteristic includes the frequency of the magnetic field.

The frequency is randomly varied over time.

In one embodiment, the system includes translation means for translating the magnetometer signal and providing a desired signal component that is representative of a change in the magnetic field.

### BRIEF DESCRIPTION OF THE FIGURES

Further features and advantages will become apparent from the following and more particular description of the present invention, as illustrated in the accompanying drawings, and in which like referenced characters generally refer to the same parts or elements throughout the views.
FIG. 1 is a schematic illustration of a physiology monitoring system, according to one embodiment of the invention.
FIG. 2 is a schematic illustration of a conventional paired magnetometer arrangement.
FIG. 3 is a side view of a subject, showing conventional positioning of the paired magnetometer arrangement shown in FIG. 2.
FIG. 4 is a perspective view of the subject, showing the positioning of magnetometers thereon in accordance with the paired magnetometer arrangement shown in FIGS. 2 and 3.
FIG. 5 is a plan view of the subject's back, showing the positioning of magnetometers thereon in accordance with the paired magnetometer arrangement shown in FIGS. 2 and 3.

### DETAILED DESCRIPTION OF THE INVENTION

Before describing the present invention in detail, it is to be understood that this invention is not limited to particularly exemplified methods, apparatuses, systems, or circuits, as such may, of course, vary. Thus, although a number of methods and systems similar or equivalent to those described herein can be used in the practice of the present invention, the preferred methods, apparatus and systems are described herein.

It is also to be understood that the terminology used herein is for the purpose of describing particular embodiments of the invention only and is not intended to be limiting.

Unless defined otherwise, all technical and scientific terms used herein have the same meaning as commonly understood by one having ordinary skill in the art to which the invention pertains.

As used in this specification and the appended claims, the singular forms "a", "an", and "the" include plural referents unless the content clearly dictates otherwise. Thus, for example, reference to "a magnetic field characteristic" includes one or more such characteristics and reference to "a magnetometer signal" includes one or more such signals.

The publications discussed herein are provided solely for their disclosure prior to the filing date of the present application. Nothing herein is to be construed as an admission that the present invention is not entitled to antedate such publication(s) by virtue of prior invention. Further, the dates of publication may be different from the actual publication dates, which may need to be independently confirmed.

### Definitions

The term "magnetometer", as used herein, means and includes a device that is adapted to generate a magnetic field or measure a magnetic field (or a characteristic associated therewith). As discussed above, magnetometers are typically employed in pairs; one magnetometer serving as a source of a magnetic field and the other magnetometer adapted to receive and measure the generated field, e.g., a characteristic associated therewith. The magnetometers disclosed herein, however, need not necessarily be paired one-to-one. For example, a magnetometer may be configured to receive transmissions from multiple magnetometers, and a single magnetometer may be configured to transmit to multiple magnetometers. Moreover, a single magnetometer may both receive and transmit signals.

As is well known in the art, paired magnetometers (e.g., a transmitter and a receiver) are responsive to a change in spaced distance therebetween. As is also well known in the art, a change in spaced distance between the paired magnetometers is generally reflected in a change in the received magnetic field.

The term "magnetometer signal", as used herein, means and includes a signal representing a measured characteristic associated with a received magnetic field, such as the strength of the generated magnetic field and/or changes thereto. Thus, in some embodiments of the invention, the signal includes the measured voltage of the receiving magnetometer (i.e., receiver). As is well known in the art, the receiver voltage is generally proportional to the strength of the received magnetic field.

The term "desired signal", as used herein, means and includes a signal or portion thereof that directly corresponds to the variable being measured. Thus, for example, in some embodiments of the invention, the desired magnetometer signal includes a signal or portion thereof that directly corresponds to a change in a received magnetic field.

The term "undesirable signal", as used herein, means and includes any signal or portion thereof that hinders accurate measurement of the variable being measured. Thus, for example, in some embodiments of the invention, an "undesirable signal" includes a signal or portion thereof that hinders accurate measurement of changes in the magnetic field of a magnetometer, including, without limitation, electromagnetic interference.

Pulmonary ventilation, tidal volume, respiratory rate, and other associated respiratory characteristics can provide a reliable and practical measure of oxygen and carbon dioxide transpiration in a living body. Respiratory characteristics are directly connected to exercise effort, physiological stress, and other physiological characteristics. One way to externally determine tidal volume is to measure the change in thoracic volume. Change in thoracic volume is caused by the expansion and contraction of the lungs. As the gas pressure in the lungs at the maxima and minima of the pressure ranges is equilibrated to surrounding air pressure, there is a very close and monotonic relationship between the volume of the lungs and the volume of air inspired.

Accurate measurement of the change in thoracic volume involves measuring the change in the diameter of the chest at the ribcage. Measurement of the change in the diameter of the chest below the ribcage can provide additional accuracy to the measurement. Monitoring changes in the diameter of the chest below the ribcage can account for diaphragm delivered breathing where the contraction and relaxation of the diaphragm muscle causes the organs of the abdomen to be pushed down and outwards, thereby increasing the available volume of the lungs.

Monitoring and analyzing respiratory characteristics can be particularly useful in athletic applications, as there is a direct link between performance and an athlete's processing of oxygen and carbon dioxide. For example, in many athletic training situations, it is helpful to know when the athlete's body transitions between aerobic exercise and anaerobic exercise, sometimes referred to as the athlete's ventilatory threshold. Crossing over the ventilatory threshold level is an indicator of pending performance limitations during sport activities. For example, it can be beneficial for athletes to train in the anaerobic state for limited periods of time. However, for many sports, proper training requires only limited periods of anaerobic exercise interrupted by lower intensity aerobic exercises. It is difficult for an athlete to determine which state, anaerobic or aerobic, he or she is in without referencing physiological characteristics such as respiratory characteristics. Therefore, respiratory monitoring and data processing can provide substantial benefits in athletic training by allowing for accurate and substantially instantaneous measurements of the athlete's exercise state. Changes in an athlete's ventilatory threshold over time, as well as patterns of tidal volume during post-exercise recovery, can be valuable to measure improvements in the athlete's fitness level over the course of a training regime. Respiratory monitoring can further allow for monitoring and analyzing changes in a subject's resting metabolic rate.

A second ventilatory threshold exists at the point when the load on the body is such that the pulmonary ventilation is no longer sufficient to support life sustainably. Dwelling too long in this state will lead to collapse and so determination of this point can be of value in medical applications, and particularly to first responders and other emergency response personnel.

The present invention includes improved methods and associated systems for limiting interference in magnetometer fields and the effects of interference on magnetic field measurement. The improved methods and associated systems substantially reduce or eliminate the drawbacks associated with prior art methods and systems for limiting interference in magnetometer fields. As set forth in detail below, in some embodiments of the invention, the transmitting magnetometers (i.e., transmitters) are adapted to generate a magnetic field having a characteristic that is randomly varied over time. In some embodiments of the invention, the transmitting magnetometers are adapted to generate a magnetic field having a characteristic that is pseudo-randomly varied over time.

As will be readily appreciated by one having ordinary skill in the art, the methods and systems of the invention provide numerous significant advantages over conventional methods and systems for limiting magnetometer interference. Among the advantages are (i) enhanced reduction of electromagnetic interference in measured magnetic fields, (ii) enhanced accuracy of measured changes in magnetic fields, and, hence, signals representing measured changes in magnetic fields, and (iii) detection of very small magnetic fields and changes thereto in high noise environments.

A further significant advantage is the accurate determination of anatomical and physiological characteristics (e.g., anatomical displacements, respiratory characteristics, etc.) based on "accurate" magnetometer signals reflecting measured changes in the magnetic fields.

Several embodiments of the methods and systems of the invention will now be described in detail. It is understood that the invention is not limited to the system and method embodiments described herein. Indeed, as will be appreciated by one having ordinary skill in the art, systems and associated methods similar or equivalent to the described systems and methods can also be employed within the scope of the present invention.

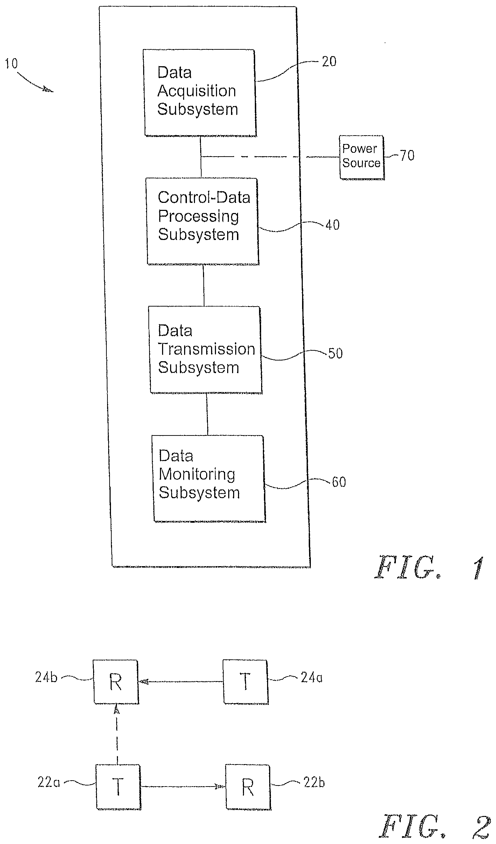

Referring first to Fig. 1, there is shown a schematic illustration of an exemplary physiology monitoring system that is adapted to (i) monitor and detect changes in (or displacements of) the anteroposterior diameters of the rib cage and abdomen, and axial displacement of the chest wall, and (ii) determine anatomical and physiological information associated with the monitored subject as a function of the magnetometer signals reflecting the noted anatomical displacements.

As illustrated in Fig. 1, the physiology monitoring system 10 includes a data acquisition subsystem 20, a control-data processing subsystem 40, a data transmission subsystem 50, a data monitoring subsystem 60, and a power source 70, such as a battery.

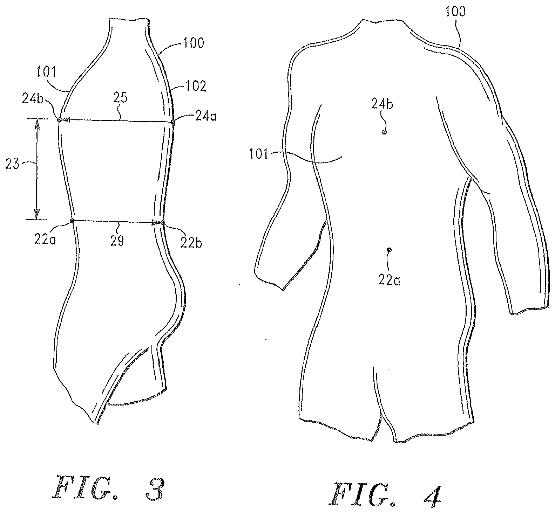

As set forth in Figs. 2 and 3, the data acquisition subsystem 20 includes paired magnetometers that are positioned on a subject 100 and adapted to monitor and detect changes in (or displacements of) the anteroposterior diameters of the rib cage and abdomen, and axial displacement of the chest wall. As illustrated in Fig. 2, the magnetometers include first transmission magnetometer 22a, first receive magnetometer 22b, second transmission magnetometer 24a, and second receive magnetometer 24b. In Fig. 2, the letter T designates the transmission magnetometers and the letter R designates the receiving magnetometers, however, the magnetometers are not limited to such designations. The magnetometers of embodiments of the present invention are described as "receiving" or "transmitting," however, each receiving coil can alternatively and independently be a transmitting coil, and each transmitting coil can alternatively and independently be a transmitting coil. Coils can also perform both receiving and transmitting functions.

Control-data processing subsystem 40 includes programs, instructions, and associated algorithms to control data acquisition subsystem 20 and, hence, the paired magnetometers, and data transmission subsystem 50 and data monitoring subsystem 60.

Control-data processing subsystem 40 is further programmed and adapted to retrieve and process magnetometer signals reflecting changes in the magnetometer fields and to determine anatomical and physiological information associated with the monitored subject (as a function of the magnetometer signals), including at least one respiratory characteristic (more preferably a plurality of respiratory characteristics).

Data monitoring subsystem 60 is designed and adapted to display physiological characteristics and parameters generated and transmitted by control-data processing subsystem 40. Control-data processing subsystem 40 is also referred to herein as "processor subsystem," "processing subsystem," and "data processing subsystem." The terms control-data processing subsystem, processor subsystem, processing subsystem, and data processing subsystem are used interchangeably in the present application.

Data transmission subsystem 50 is programmed and adapted to monitor and control the communication links and, hence, transmissions by and between data acquisition subsystem 20, control-data processing subsystem 40, and data monitoring subsystem 60.

Further details of the noted physiological monitoring system are set forth in U.S. Provisional Application No. 61/275,576, filed September 1, 2009, co-pending U.S. Application No. 12/869,592, filed concurrently herewith, U.S. Provisional Application No. 61/275,575, filed September 1, 2009, and co-pending U.S. Application No. 12/869,582, filed concurrently herewith.

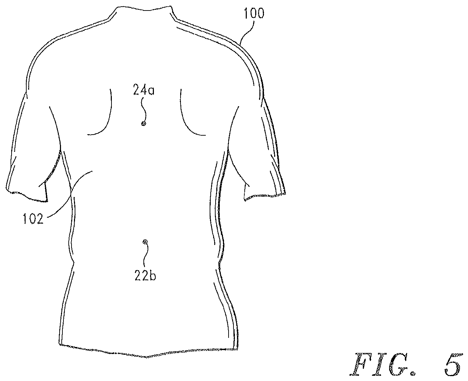

As will be readily appreciated by one having ordinary skill in the art, the paired magnetometers can be disposed in various anatomically appropriate positions on a subject to monitor and measure the change in distance (or displacement) between the magnetometers. Referring now to Figs. 3-5, there is shown the positioning of magnetometers 22a, 22b, 24a, 24b on a subject or patient 100, in accordance with one embodiment of the invention.

As illustrated in Figs. 3-5, first transmission magnetometer (i.e., first transmitter) 22a is preferably positioned on front 101 of subject 100 proximate the umbilicus of subject 100, and first receive magnetometer (i.e., first receiver) 22b is preferably positioned proximate the same axial position, but on back 102 of the subject 100. Second receive magnetometer (i.e., second receiver) 24b is preferably positioned on front 101 of subject 100 proximate the base of the sternum, and second transmission magnetometer (i.e., second transmitter) 24a is positioned proximate the same axial position, but on back 102 of the subject 100.

As set forth in co-pending U.S. Patent Application No. 12/231,692, the positions of transmission magnetometers 22a, 24a and receive magnetometers 22b, 24b can be reversed (i.e., transmission magnetometer 22a and receive magnetometer 24b can be placed on back 102 of subject 100 and transmission magnetometer 24a and receive magnetometer 22b can be placed on front 101 of subject 100. Both transmission magnetometers 22a and 24a can also be placed on front 101 or back 102 of subject 100 and receive magnetometers 22b and 24b can be placed on the opposite side.

As subject or patient 100 breathes, displacement(s) of the rib cage and abdomen (i.e., changes in the distance between paired magnetometers 22a, 22b and 24a, 24b, denoted, respectively, by arrow 29 and arrow 25 in Fig. 3) is determined from measured changes in the magnetic field between paired magnetometers 22a, 22b and 24a, 24b. The axial displacement of the chest wall, denoted by arrow 23, (e.g., xiphi-umbilical distance (Xi)), is also determined from measured changes in the magnetic field between magnetometers 22a and 24b, magnetometer 24b being a dual-function electromagnetic coil, where "dual function coil" refers to a coil capable of receiving transmissions from a plurality of different transmission coils (thus, magnetometer 24b is adapted to receive magnetic field transmissions from magnetometers 22a and 24a).

As indicated above, the measured displacements are typically employed to determine anatomical and physiological information associated with the monitored subject, including, at least one or more respiratory characteristics. As set forth in U.S. Provisional Application No. 61/275,575, filed September 1, 2009, and co-pending U.S. Application No. 12/869,582, filed concurrently herewith, additional paired magnetometers can also be employed, and the multiple measured displacements can be employed to assess additional anatomical and physiological characteristics, such as determining and characterizing the relationship(s) of chest wall movement(s) to respiratory activity and respiratory associated events, such as speaking, sneezing, laughing and coughing.

As also indicated above, the magnetometers (e.g., magnetometers 22a, 22b, 24a, 24b) are, however, susceptible to interference from environmental sources (e.g., power lines, fluorescent bulbs, electric motors, etc.) that have a frequency synchronous with or sub-harmonic of the magnetic field frequency.

The spurious interference can, and in many instances will, result in inaccurate measurements of the magnetometer field. Anatomical displacements determined from the inaccurate magnetic field measurements (e.g., magnetometer signals reflecting anatomical displacements) and, hence, respiratory characteristics derived therefrom are, thus, likely to be inaccurate.

Applicant has, however, found that providing magnetometers that provide magnetic fields having a characteristic that randomly or pseudo-randomly varies over time substantially reduces and, in many instances, eliminates the effects of the spurious, synchronous, and/or sub-harmonic interference on magnetic field measurement. In some embodiments of the invention, the magnetic field characteristic includes the frequency of the magnetic field. In some embodiments, the magnetic field characteristic includes the frequency period of the magnetic field.

In some embodiments of the invention, the magnetic field frequency is randomly varied over time. According to the invention, the randomly varied frequency is, however, never greater than 10,000 Hz.

Thus, in the noted embodiments, one of the paired magnetometers (i.e., a transmitter) is adapted to provide a magnetic field having a frequency that is randomly varied over time, and the other magnetometer (i.e., a receiver) is adapted to receive and measure the generated magnetic field.

In one embodiment of the invention, the frequency of the magnetic field is randomly varied between approximately 8000 and 9999 Hz. In another embodiment, the frequency of the magnetic field is randomly varied between approximately 8900 and 9100 Hz.

In some embodiments, the frequency period of the magnetic field is pseudo-randomly varied. In the noted embodiments, one of the paired magnetometers (i.e., a transmitter) is accordingly adapted to provide a magnetic field having a frequency period that is pseudo-randomly varied, and the other magnetometer (i.e., a receiver) is adapted to receive and measure the generated magnetic field.

In one embodiment of the invention, the period of the magnetic field is pseudo-randomly varied between approximately 1/8000 and 1/9999 seconds. In another embodiment, the period of the magnetic field is pseudo-randomly varied between approximately 1/ 8900 and 1/9100 seconds.

According to the invention, the magnetic field frequency can also be pseudo-randomly varied between approximately 8000 and 9999 Hz. The frequency period can also be randomly varied between approximately 1/8000 and 1/9999 seconds.

One of the paired magnetometers (i.e., a transmitter) can also be adapted to provide a magnetic field having at least one characteristic that is randomly varied over time and at least one characteristic that is pseudo-randomly varied over time, and the other magnetometer (i.e., a receiver) can be adapted to receive the generated magnetic field.

According to the invention, various conventional means can be employed to provide the random and pseudo-random varied magnetic fields. Such means include a generator, such as, for example, a conventional random or pseudo-random number generator, a shift register with selected circuitry and a seed number, a random noise generator that is based on the thermal noise existing in suitable electronic devices, such as noise diodes, and an appropriately programmed microprocessor.

In a preferred embodiment of the invention, the random and pseudo-random varied magnetic fields are provided by a time varying magnetic field generator, e.g., a random or pseudo-random number generator that is operatively connected to a transmission magnetometer, for example magnetometer 22a, whereby the magnetometer generates a magnetic field having a frequency, or frequency period, that is randomly (or pseudo-randomly) varied over time.

In some embodiments of the invention, a conventional spread spectrum technique is employed to extract the desired signal and/or component(s) thereof from periodic interference (or noise) or from random and pseudo-random noise that is uncorrelated with the measurement signal.

In some embodiments, translation circuitry is employed to provide the desired signal that is representative of the magnetic field and, hence, changes thereto.

### EXAMPLES

The following examples are provided to enable those skilled in the art to more clearly understand and practice the present invention. They should not be considered as limiting the scope of the invention, but merely as being illustrative and representative thereof.

### Example 1

A physiology monitoring system, such as that illustrated in Fig. 1, is provided. Paired magnetometers 22a, 22b, and 24a, 24b are positioned on a subject, as shown in Figs. 3-5 above.

The magnetic field between one of the magnetometer pairs (e.g., magnetometers 22a, 22b) has a steady state frequency of approximately 9000 Hz. The magnetic field between the other pair of magnetometers (e.g., magnetometers 24a, 24b) has a steady state frequency of approximately 8760 Hz.

Disposed close to the monitored subject is a fluorescent light generating a signal having frequencies in the range of 8800 to 9000 Hz. Interference resulting from the fluorescent light signal is detected in the magnetic field between magnetometers 22a and 22b and, hence, in signals representing measured changes in the magnetic field.

The frequency of the magnetic fields between the paired magnetometers 22a-22b and 24a-24b is then randomly varied between 8900 and 9100 Hz and 8660 and 8860 Hz, respectively, by a random number generator. The effect of the interference is substantially minimized (i.e., approx. 1/200 of the original effect).

### Example 2

In example 2, the same physiological monitoring system is employed and the same conditions are present. However, in example 2, the frequency period of the magnetic fields between the paired magnetometers 22a-22b and 24a-24b is pseudo-randomly varied between 1/8900 and 1/9100 seconds and 1/8660 and 1/8860 seconds, respectively. Interference by the fluorescent light signal is similarly minimized.

The methods and systems of the invention, described above, thus provide numerous significant advantages over conventional methods and systems for limiting magnetometer interference. Among the advantages are (i) enhanced reduction of electromagnetic interference in measured magnetic fields, (ii) enhanced accuracy of measured changes in magnetic fields, and, hence, signals representing same, and (iii) detection of very small magnetic fields and changes thereto in high noise environments.

A further significant advantage is the accurate determination of anatomical and physiological characteristics (e.g., anatomical displacements, respiratory characteristics, etc.) based on "accurate" magnetometer signals reflecting measured changes in the magnetic fields.

Additional advantages and applications of the present invention are apparent with reference to the systems and methods disclosed in U.S. Patent Application No. 12/869,578, filed concurrently herewith, U.S. Patent Application No. 12/869,582, filed concurrently herewith, U.S. Patent Application No. 12/869,585, filed concurrently herewith, U.S. Patent Application No. 12/869,592, filed concurrently herewith, U.S. Patent Application No. 12/869,627, filed concurrently herewith, U.S. Patent Application No. 12/869,625, filed concurrently herewith, and U.S. Patent Application No. 12/869,586, filed concurrently herewith.

Without departing from the scope of this invention, one of ordinary skill can make various changes and modifications to the invention to adapt it to various usages and conditions. As such, these changes and modifications are properly, equitably, and intended to be, within the full range of equivalence of the following claims.

## Claims

1. A device for generating a magnetic field for athletic performance monitoring, comprising:
a first pair of magnetometers (22a, 22b) and a second pair of magnetometers (24a, 24b) positioned on an athlete (100), wherein one magnetometer (22a, 24a) of each pair of magnetometers is configured to transmit an external magnetic field and another one magnetometer (22b, 24b) of each pair of magnetometers is configured to receive and monitor the magnetic field;
a generator operatively connected to the one magnetometers (22a, 24a) and configured to vary a characteristic of the external magnetic field, wherein the characteristic is varied over time, wherein the another one magnetometers (22b, 24b) of each pair of magnetometers are configured to generate at least one magnetometer signal representing a change in the magnetic field;
**characterized in that:**
the generator is configured to randomly or pseudo-randomly vary the frequency of the first pair of magnetometers (22a, 22b) between 8900 and 9100 Hz and to randomly or pseudo-randomly vary the frequency of the second pair of magnetometers (24a, 24b) between 8660 and 8860 Hz.

2. The device of claim 1, wherein the generator includes a random or pseudo-random number generator.

3. The device of claim 1, wherein the generator includes a random or pseudo-random noise generator.

4. The device of claim 1, further comprising translation circuitry configured to translate the magnetometer signal and to provide a signal component that is representative of a change in the magnetic field.

5. A method of operating a device of any of the preceding claims to generate a magnetic field for athletic performance monitoring.

## Patentansprüche

1. Vorrichtung zum Erzeugen eines Magnetfeldes zur Überwachung von sportlichen Leistungen, umfassend:
ein erstes Paar Magnetometer (22a, 22b) und ein zweites Paar Magnetometer (24a, 24b), die auf einem Athleten (100) positioniert sind, wobei ein Magnetometer (22a, 24a) jedes Paares von Magnetometern konfiguriert ist, um ein externes Magnetfeld zu übertragen, und ein weiteres Magnetometer (22b, 24b) jedes Paares von Magnetometern konfiguriert ist, um das Magnetfeld zu empfangen und zu überwachen;
einen Generator, der funktionsfähig mit den einen Magnetometern (22a, 24a) verbunden und konfiguriert ist, um eine Eigenschaft des äußeren Magnetfeldes zu variieren, wobei die Eigenschaft im Laufe der Zeit variiert wird, wobei die anderen einen Magnetometer (22b, 24b) jedes Paares von Magnetometern konfiguriert sind, um mindestens ein Magnetometersignal zu erzeugen, das eine Änderung des Magnetfeldes darstellt;
**dadurch gekennzeichnet, dass**:
der Generator konfiguriert ist, um die Frequenz des ersten Paares von Magnetometern (22a, 22b) zwischen 8900 und 9100 Hz zufällig oder pseudozufällig zu variieren und um die Frequenz des zweiten Paares von Magnetometern (24a, 24b) zwischen 8660 und 8860 Hz zufällig oder pseudozufällig zu variieren.

2. Die Vorrichtung nach Anspruch 1, wobei der Generator einen Zufalls- oder Pseudozufallszahlengenerator beinhaltet.

3. Die Vorrichtung nach Anspruch 1, wobei der Generator einen Zufalls- oder Pseudozufallsrauschgenerator beinhaltet.

4. Die Vorrichtung nach Anspruch 1, ferner umfassend eine Übersetzungsschaltung, die konfiguriert ist, um das Magnetometersignal zu übersetzen und eine Signalkomponente bereitzustellen, die für eine Änderung des Magnetfeldes repräsentativ ist.

5. Ein Verfahren zum Betreiben einer Vorrichtung nach einem der vorhergehenden Ansprüche, um ein Magnetfeld für eine Überwachung von sportlichen Leistungen zu erzeugen.

## Revendications

1. Un dispositif pour la génération d'un champ magnétique destiné à la surveillance de performances athlétiques, comprenant :
une première paire de magnétomètres (22a, 22b) et une seconde paire de magnétomètres (24a, 24b) positionnés sur un athlète (100), l'un des magnétomètres (22a, 24a) de chaque paire de magnétomètres étant configuré pour émettre un champ magnétique externe et un autre magnétomètre (22b, 24b) de chaque paire de magnétomètres étant configuré pour recevoir et suivre le champ magnétique ;
un générateur relié de manière opérante auxdits un magnétomètre (22a, 24a) et configuré de manière à modifier une caractéristique du champ magnétique externe, la caractéristique étant modifiée dans le temps, ledit autre magnétomètre (22b, 24b) de chaque paire de magnétomètres étant configuré pour générer au moins un signal magnétométrique représentant un changement du champ magnétique ;
**caractérisé en ce que** :
le générateur est configuré pour modifier de façon aléatoire ou pseudoaléatoire la fréquence de la première paire de magnétomètres (22a, 22b) entre 8900 et 9100 Hz et pour modifier de façon aléatoire ou pseudoaléatoire la fréquence de la seconde paire de magnétomètres (24a, 24b) entre 8660 et 8860 Hz.

2. Le dispositif de la revendication 1, dans lequel le générateur comprend un générateur de nombres aléatoires ou pseudoaléatoires.

3. Le dispositif de la revendication 1, dans lequel le générateur comprend un générateur de bruit aléatoire ou pseudoaléatoire.

4. Le dispositif de la revendication 1, comprenant en outre une circuiterie de traduction configurée pour traduire le signal magnétométrique et délivrer une composante de signal qui est représentative d'un changement du champ magnétique.

5. Un procédé de mise en oeuvre d'un dispositif selon l'une des revendications précédentes pour générer un champ magnétique pour le suivi de performances athlétiques.
